# EUROPÄISCHE PATENTSCHRIFT

(11) **EP 0 645 366 B1**
(45) Veröffentlichungstag und Bekanntmachung des Hinweises auf die Patenterteilung: **20.05.1998**
(21) Anmeldenummer: 94113885.1
(22) Anmeldetag: 05.09.1994
(51) Int. Cl.: C07C 69/88, C07C 69/92, C07C 67/10

(54) **Verfahren zur Herstellung von 3-Hydroxy-2,4,5-trifluorbenzoesäurealkylestern und/oder 3-Alkoxy-2,4,5-trifluorbenzoesäurealkylestern**
Process for the preparation of alkyle esters of 3-hydroxy-2,4,5-trifluoro benzoic acid and/or of 3-alcoxy-2,4,5 trifluoro benzoic acid
Procédé de préparation d'esters alkyliques de l'acide 3-hydroxy-2,4,5-trifluorobenzoique et/ou de l'acide 3-alcoxy-2,4,5-trifluorobenzoique

(30) Priorität: 18.09.1993 DE 4331799
(43) Veröffentlichungstag der Anmeldung: 29.03.1995
(73) Patentinhaber: Clariant GmbH, 65929 Frankfurt am Main (DE)
(72) Erfinder: Pfirmann, Ralf, Dr., D-64347 Griesheim (DE); Papenfuhs, Theodor, Dr., D-60433 Frankfurt am Main (DE)

(56) Entgegenhaltungen:
- US-A- 4 513 146

## Beschreibung

Die vorliegende Erfindung betrifft ein vorteilhaftes Verfahren zur Herstellung von 3-Hydroxy-2,4,5-trifluorbenzoesäurealkylester und/oder 3-Alkoxy-2,4,5-trifluorbenzoesäurealkylester. Die vorstehend genannten Stoffe, insbesondere 3-Hydroxy-2,4,5-trifluorbenzoesäuremethylester und 3-Methoxy-2,4,5-trifluorbenzoesäuremethylester stellen, ebenso wie die durch die Hydrolyse von 3-Methoxy-2,4,5-trifluorbenzoesäurealkylester herstellbare 3-Methoxy-2,4,5-trifluorbenzoesäure, wichtige Vorprodukte zur Herstellung von antibakteriell wirksamen Mitteln aus der Reihe der Fluorchinoloncarbonsäuren dar.

Die Überführung von 3-Alkoxy-2,4,5-trifluorbenzoesäuren in antibakteriell wirksame Mittel läßt sich nach literaturbekannten Methoden über mehrere Stufen (EOS 241 206 und EOS 230 295) durchführen. 3-Methoxy-2,4,5-trifluorbenzoesäuremethylester kann ebenfalls zur Synthese von antibakteriellen Mitteln eingesetzt werden. Seine Umwandlung in weitere für diese Synthese benötigte Vorstufen gelingt nach US 5 047 538. Zur Herstellung von speziellen, insbesondere arylierte Alkylgruppen enthaltenden, in 3-Stellung substituierten 2,4,5-Trifluorbenzoesäuren, die als Vorprodukte für die Herstellung antibakterieller Mittel dienen, werden entsprechende 3-Hydroxy-2,4,5-trifluorbenzoesäurealkylester, insbesondere 3-Hydroxy-2,4,5-trifluorbenzoesäuremethylester (JP 04 230 344), benötigt.

3-Methoxy-2,4,5-trifluorbenzoesäure läßt sich durch Methylierung von 3-Hydroxy-2,4,5-trifluorbenzoesäure mittels Dimethylsulfat (JP 03 232 838, JP 01 268 662) oder durch Decarboxylierung von 3,5,6-Trifluor-4-methoxyphthalsäure (JP 03 279 348) mit Basen in dipolar aprotischen Lösungsmitteln herstellen. Die Alkylierung (Methylierung) mittels eines Dialkylsulfates (Dimethylsulfat) verläuft im wesentlichen zwar glatt, stellt aber eine technische Realisierung wegen der sehr hohen Toxizität und Karzinogenität von Dialkylsulfaten (Dimethylsulfat) vor erhebliche Probleme. Zudem verläuft die Alkylierung (Methylierung) von 3-Hydroxy-2,4,5-trifluorbenzoesäure wegen der hohen Reaktivität von Di-Alkylsulfat (Dimethylsulfat) vergleichsweise unkontrolliert ab, was dazu führt, daß die Umsetzung sich nicht, wie gewünscht, selektiv durchführen läßt.

Die Decarboxylierung von 3,5,6-Trifluor-4-methoxy-phthalsäure verläuft zwar ebenfalls glatt, ihre Selektivität läßt sich jedoch nicht so steuern, daß ausschließlich die gewünschte 3-Methoxy-Verbindung entsteht. Als unerwünschtes Nebenprodukt bildet sich stets die schwer abzutrennende 4-Methoxy-2,3,5-trifluorbenzoesäure, die anschließend in der weiteren Synthese des Wirkstoffes mitgeschleppt wird und gegebenenfalls toxische Verunreinigungen in der antibakteriellen Substanz verursachen kann.

3-Methoxy-2,4,5-trifluorbenzoesäuremethylester läßt sich durch eine vollständige Methylierung von 3-Hydroxy-2,4,5-trifluorbenzoesäure mittels Dimethylsulfat nach Standardmethoden oder durch Veresterung von 3-Methoxy-2,4,5-triflurobenzoesäure mittels Alkoholen unter Verwendung saurer Katalysatoren herstellen.

Der vorliegenden Erfindung liegt die Aufgabe zugrunde, ein einfaches Verfahren zur Herstellung von Alkylierungsprodukten der 3-Hydroxy-2,4,5-trifluorbenzoesäure bereitzustellen, das die gewünschten Produkte nicht nur in hoher Ausbeute und Reinheit zugänglich macht, sondern auch den Einsatz hoch toxischer Stoffe entbehrlich macht und die Entstehung unerwünschter Abwässer, die eine Belastung der Umwelt darstellen, vermeidet.

Diese Aufgabe wird gelöst durch ein Verfahren zur Herstellung von 3-Hydroxy-2,4,5-trifluorbenzoesäurealkylester und/oder 3-Alkoxy-2,4,5-trifluorbenzoesäurealkylester. Es ist dadurch gekennzeichnet, daß man 3-Hydroxy-2,4,5-trifluorbenzoesäure bei 80 bis 200 °C, gegebenenfalls in Anwesenheit wenigstens eines Katalysators, mit einem Dialkylcarbonat umsetzt.

Durch das erfindungsgemäße Verfahren gelangt man zu Verbindungen, die an der Carboxy-Gruppe und/oder Hydroxy-Gruppe alkyliert sind. Als Ausgangsmaterial für die Synthese dient 3-Hydroxy-2,4,5-trifluorbenzoesäure, die ihrerseits ein Vorprodukt zur Herstellung von antibakteriell wirksamen Stoffen darstellt und nach bekannten Verfahren aus Tetrafluorphthalsäure durch Austausch eines Fluoratoms gegen eine Hydroxy-gruppe und anschließende Decarboxylierung der entsprechenden Hydroxy-Verbindung hergestellt werden kann. Im Gegensatz zu Dialkylsulfaten, insbesondere Dimethylsulfat, besitzen Dialkylcarbonate, insbesondere Dimethylcarbonat, eine wesentlich geringere Toxizität, was möglicherweise darauf zurückzuführen ist, daß Dialkylcarbonate bzw. Dimethylcarbonat bei Normalbedingungen, also bei Normaldruck und Normaltemperatur, kaum eine methylierende Wirkung aufweisen.
Eine Gegenüberstellung der toxikologischen Daten von Dimethylcarbonat, als Beispiel für ein Dialkylcarbonat, und Dimethylsulfat, als Vertreter eines Dialkylsulfates, zeigt gravierende Unterschiede. So beträgt die LD₅₀ von Dimethylcarbonat (Ratte, oral) 12800 mg/kg Körpergewicht, hingegen liegt der entsprechende Wert für Dimethylsulfat mit 440 mg/kg Körpergewicht nahezu um den Faktor 30 niedriger, d.h. die Toxizität ist entsprechend höher. Die entsprechende LD₅₀ für subkutane Injektion beträgt bei Dimethylcarbonat 8500 mg/kg Körpergewicht, dagegen bei Dimethylsulfat nur 30 mg/kg Körpergewicht.
Wird Dimethylcarbonat von einer Ratte inhaliert, so bemerkt man bei Konzentrationen bis zu 1000 ppm in 6 Stunden keinerlei Vergiftungserscheinungen, wird die Dosis auf 5000 ppm erhöht, werden Effekte sichtbar, die jedoch in unbelasteter Atmosphäre rasch abklingen. Demgegenüber wirkt Dimethylsulfat bereits in einer Konzentration von lediglich 30 ppm innerhalb von 4 Stunden tödlich. Darüber hinaus weist Dimethylsulfat teratogene, mutagene und karzinogene Wirkungen auf. Von Dimethylcarbonat hingegen sind bislang keine chronischen Wirkungen bekannt.

Ein weiterer wesentlicher Unterschied bei der Verwendung von Dialkylcarbonaten einerseits und Dialkylsulfaten andererseits besteht darin, daß die Umsetzung mittels Dialkylcarbonaten zur Entstehung von Alkoholen und Kohlendioxid als Abfallprodukten führt, während bei der Umsetzung mit Dialkylsulfaten lediglich eine Alkylgruppe zur Alkylierung ausgenutzt wird und wasserlösliche Alkylsulfate als Abfallprodukte entstehen. Da die Umsetzungen mit Dialkylsulfaten üblicherweise in Gegenwart von Wasser durchgeführt werden, fällt dementsprechend stets ein mit Alkylsulfaten stark belastetes Abwasser an.

Ein besonderer Vorteil des erfindungsgemäßen Verfahrens besteht darin, die Umsetzung von 3-Hydroxy-2,4,5-trifluorbenzoesäure mit dem Dialkylcarbonat so steuern zu können, daß entweder der entsprechende 3-Hydroxy-2,4,5-trifluorbenzoesäurealkylester oder der entsprechende 3-Alkoxy-2,4,5-trifluorbenzoesäurealkylester entsteht. Die Umsetzung führt in beiden Fällen mit hoher Selektivität zu dem jeweils gewünschten Produkt. Es lassen sich jedoch auch Mischungen beider Stoffe herstellen.

Ein weiterer Vorteil des erfindungsgemäßen Verfahrens ist darin zu sehen, daß die Umsetzung in wasserfreiem Medium unter neutralen Bedingungen, d.h. ohne Zusatz eines sauren oder basischen Katalysators, durchgeführt werden kann. Korrosionsprobleme, wie sie bei Verwendung von wäßrigen Mineralsäuren oder wäßrigen Basen zu erwarten sind, treten nicht auf. Der Verzicht auf derartige saure oder basische Katalysatoren vereinfacht die Durchführbarkeit des Verfahrens erheblich.

Beabsichtigt man den 3-Hydroxy-2,4,5-trifluorbenzoesäurealkylester herzustellen, so führt man die Umsetzung bei vergleichweise niedrigen Temperaturen durch, will man hingegen den 3-Alkoxy-2,4,5-trifluorbenzoesäurealkylester herstellen, so läßt man die Reaktion bei höheren Temperaturen ablaufen.

Diese beiden direkt durch Alkylierung herstellbaren Verbindungen - 3-Hydroxy-2,4,5-trifluorbenzoesäurealkylester einerseits und 3-Alkoxy-2,4,5-trifluorbenzoesäurealkylester andererseits - lassen sich auf einfache Art und Weise durch eine fraktionierte Destillation reinigen, was im Falle der als Einsatzmaterial dienenden 3-Hydroxy-2,4,5-trifluorbenzoesäure nicht und im Falle von 3-Alkoxy-trifluorbenzoesäuren im allgemeinen nur unter erheblichen Schwierigkeiten möglich ist. Auf diese Weise kann man zu Produkten von sehr hoher Reinheit gelangen, was ansonsten nicht ohne weiteres gelingt.

Das Ergebnis der Alkylierung (Methylierung) ist als überraschend anzusehen, da aus dem Stand der Technik (M. Lissel et al., Kontakte Darmstadt) 1990 (1), 20-23 und dort zitierte Literatur) hervorgeht, daß die Alkylierung einer Carbonsäure-Gruppe üblicherweise bei wesentlich höheren Temperaturen als die Alkylierung einer Phenol-Gruppe erfolgt. Lissel et al. geben für die Alkylierung von Phenolen und Carbonsäuren einen Temperaturunterschied von 60 °C an.

Man führt die Umsetzung im allgemeinen in einem Temperaturbereich von 80 bis 200 °C durch.
Für eine Vielzahl von Fällen hat es sich als nützlich erwiesen, die Umsetzung bei 120 bis 195 °C, insbesondere 135 bis 190 °C durchzuführen. Will man 3-Hydroxy-2,4,5-trifluorbenzoesäurealkylester herstellen, so läßt man 3-Hydroxy-2,4,5-trifluorbenzoesäure üblicherweise bei 80 bis 165 °C mit dem Dialkylcarbonat reagieren. In einer Reihe von Fällen hat es sich als günstig erwiesen, diese Umsetzung (Monoalkylierung) bei 120 bis 165, insbesondere bei 135 bis 160 °C durchzuführen.

Beabsichtigt man 3-Alkoxy-2,4,5-trifluorbenzoesäurealkylester herzustellen, so setzt man die 3-Hydroxy-2,4,5-trifluorbenzoesäure üblicherweise bei 145 bis 200 °C um. In einer Vielzahl von Fällen hat es sich als vorteilhaft erwiesen, diese Umsetzung (Dialkylierung) bei 145 bis 195, insbesondere 150 bis 190 °C durchzuführen.
Durch Wahl geeigneter Verfahrensbedingungen lassen sich auch Mischungen, die 3-Hydroxy-2,4,5-trifluorbenzoesäurealkylester und 3-Alkoxy-2,4,5-trifluorbenzoesäurealkylester in beliebigem Verhältnis enthalten, herstellen.

Um den Ablauf der Reaktion günstig zu gestalten, empfiehlt es sich in vielen Fällen, einen Katalysator zu verwenden.
Geeignet als Katalysator ist eine aus einem Metalljodid und einem Alkylierungskatalysator bestehende Mischung.
Gut geeignet als Alkylierungskatalysator ist 4-Dimethylaminopyridin oder ein auf einem festen Träger fixiertes 4-Dimethylaminopyridin. Besonders einfach gestaltet sich die erfindungsgemäße Umsetzung bei Verwendung von 4-Dimethylaminopyridin.

Überraschend ist, daß trotz der Anwendung katalytischer Mengen an Base (Alkylierungskatalysator) der aus der Umsetzung des Dialkylcarbonats resultierende Alkohol keine nucleophile Substitution eines Fluoratoms im Austausch gegen eine Alkoxygruppe verursacht.

Als Metalljodid eigen sich Kaliumjodid, Natriumjodid, Rubidiumjodid oder Cäsiumjodid, insbesondere Natriumjodid oder Kaliumjodid, bevorzugt Kaliumjodid. Es lassen sich auch Mischungen dieser Jodide verwenden.

In einer Reihe von Fällen erweist es sich als günstig, zusätzlich einen weiteren Katalysator (Co-Katalysator) zu verwenden. Gut geeignet als Co-Katalysator sind Phasentransferkatalysatoren.
Geeignet als Phasentransferkatalysatoren sind Kronen-Ether, insbesondere 18-Krone-6 oder Dibenzo-18-Krone-6. Es lassen sich auch Mischungen von Phasentransferkatalysatoren einsetzen.

Man setzt das Metalljodid üblicherweise in einer Menge von 0,1 bis 5, insbesondere 0,25 bis 2 Mol-%, bezogen auf 3-Hydroxy-2,4,5-trifluorbenzoesäure ein.

Der Phasentransferkatalysator (Co-Katalysator) wird in einer Menge von 0,1 bis 5, insbesondere 0,25 bis 2 Mol-%, bezogen auf 3-Hydroxy-2,4,5-trifluorbenzoesäure, eingesetzt.

Es hat sich als nützlich erwiesen, einen Katalysator zu verwenden, der das Metalljodid und den Alkylierungskatalysator in einem bestimmten Verhältnis enthält. Üblicherweise enthält der Katalysator je Mol Metalljodid 1 bis 5, insbesondere 2 bis 3 Mol Alkylierungskatalysator.

Die Umsetzung kann ohne Katalysator durchgeführt werden, man muß hierbei jedoch eine geringere Umsetzungsgeschwindigkeit in Kauf nehmen.

Zur Durchführung der Umsetzung setzt man je Mol 3-Hydroxy-2,4,5-trifluorbenzoesäure 1 bis 100, insbesondere 1,5 bis 50, bevorzugt 5 bis 30 Mol Dialkylcarbonat ein. Im Falle einer Monoalkylierung kommt man im allgemeinen mit geringeren Mengen Dialkylcarbonat aus, während im Falle einer Dialkylierung üblicherweise größere Mengen Dialkylcarbonat erforderlich sind.

Als Dialkylcarbonate eignen sich solche, die 1 bis 8, insbesondere 1 bis 4 Kohlenstoffatome je Alkylgruppe enthalten.
Als Dialkylcarbonat kommen Dimethylcarbonat, Diethylcarbonat, Di-n-propylcarbonat oder Di-n-butylcarbonat in Betracht. Gut geeignet als Dialkylcarbonat sind Dimethylcarbonat oder Diethylcarbonat, insbesondere Dimethylcarbonat.

Falls gewünscht, kann man das erfindungsgemäße Verfahren auch in Anwesenheit eines inerten Lösungsmittels durchführen. Als inertes Lösungsmittel kann man einen aliphatischen Kohlenwasserstoff, einen aromatischen Kohlenwasserstoff, einen chlorierten aromatischen Kohlen-Wasserstoff, ein dipolar aprotisches Lösungsmittel oder Mischungen dieser Lösungsmittel einsetzen. Als Beispiel für einen aliphatischen Kohlenwasserstoff sind Hexan, Heptan oder Oktan, als Beispiele für einen aromatischen Kohlen- Wasserstoff Toluol oder Xylol, als Beispiel für einen chlorierten aromatischen Kohlenwasserstoff, Chlorbenzol, Dichlorbenzol oder Chlortoluol und als Beispiel für ein dipolar aprotisches Lösungsmittel sind Tetrahydrofuran, Sulfolan, Dioxan oder N,N-Dimethylacetamid zu nennen.

Es ist allerdings auch möglich, auf die Zugabe eines inerten Lösungsmittels zu verzichten.

Besonders einfach gestaltet sich das erfindungsgemäße Verfahren, indem man überschüssig eingesetztes Dialkylcarbonat als Lösungsmittel verwendet.

Nach Beendigung der Reaktion destilliert man üblicherweise das Lösungsmittel bzw. überschüssig eingesetztes Dialkylcarbonat ab und kristalliert das im Sumpf verbleibende Produkt um. Bei Verwendung eines rohen, schwer zu reinigenden Ausgangsmaterials erweist es sich als besonders vorteilhaft, das im Sumpf befindliche Wertprodukt entweder durch eine einfache Destillation über Kopf vorzureinigen und dann gegebenenfalls umzulösen (umzukristallisieren) oder die Reindarstellung in einem einzigen Schritt durch fraktionierte Destillation auf den gewünschten Reinheitsgrad vorzunehmen.

Das erfindungsgemäße Verfahren kann bei Normaldruck, Unterdruck oder Überdruck durchgeführt werden, wobei eine Arbeitsweise bei Normaldruck oder Überdruck bis etwa 1,0, insbesondere 0,5 MPa bevorzugt wird. Üblicherweise führt man die Umsetzung bei einem konstanten Druck bis etwa 0,5 MPa durch und entfernt mittels einer Druckhaltevorrichtung das bei dieser Reaktion entstehende Gas (Kohlendioxid) kontinuierlich.

Die nachfolgenden Beispiele belegen die Erfindung, ohne sie beschränken.

### Experimenteller Teil

### Beispiel 1

Man löst unter leichtem Erwärmen 19,2 g (0,1 Mol) 3-Hydroxy-2,4,5-trifluorbenzoesäure in 100 g Dimethylcarbonat, überführt die Mischung in einen mit PTFE (Polytetrafluorethylen) ausgekleideten Autoklaven, setzt 0,5 g Kaliumjodid und 0,25 g 4-Dimethylaminopyridin zu, erhitzt unter Rühren auf 150 °C und läßt 16 Stunden reagieren. Nach Abkühlen weist der Autoklav noch einen Druck von 1 MPa auf.
Die zu Beginn der Reaktion gelb-orangefarbene Lösung ist nach Abschluß der Umsetzung dunkelrot.
Das Reaktionsgemisch weist nach gaschromatographischer Analyse neben Dimethylcarbonat 89 GC-Flächenprozent 3-Hydroxy-2,4,5-trifluorbenzoesäuremethylester und 11 GC-Flächenprozent 3-Methoxy-2,4,5-trifluorbenzoesäuremethylester (identifiziert durch GC-MS) auf. Nach vollständiger Abtrennung von Dimethylcarbonat mittels eines Rotationsverdampfers werden 20,4 g Produktgemisch als rotes Öl erhalten.

### Beispiel 2

Man löst unter Rühren 0,25 g Kaliumjodid, 0,5 g 4-Dimethylaminopyridin und 51 g rohe 3-Hydroxy-2,4,5-trifluorbenzoesäure (Gehalt 40,3 g 3-Hydroxy-2,4,5-trifluorbenzoesäure, ermittelt durch HPLC, korrigiert um response-Faktoren) in 250 g Dimethylcarbonat, überführt die Mischung in einen mit PTFE ausgekleideten Autoklaven, erhitzt unter Rühren auf 145 °C und läßt 16 Stunden reagieren. Anschließend kühlt man ab und entspannt.
Die dem Autoklaven entnommene Lösung wird von unlöslichem Rückstand abfiltriert und das Dimethylcarbonat abdestilliert. Die anfallende Mischung enthält < 1 % 3-Methoxy-2,4,5-trifluorbenzoesäuremethylester. Durch Destillation (1 mbar = 0,1 kPa) über einen Spritzschutz erhält man 36 g schwach gelb gefärbtes Öl (Kopftemperatur 107 bis 115 °C). Dieses Produkt wird mit 200 g heißen Wasser ausgerührt. Nach Abkühlen und Abtrennen erhält man 33,6 g (0,163 Mol) 3-Hydroxy-2,4,5-trifluorbenzoesäuremethylester (78 % Ausbeute; Reingehalt nach GC/HPLC > 99 %).

### Beispiel 3

Man löst unter Rühren 38,4 g (0,2 Mol) 3-Hydroxy-2,4,5-trifluorbenzoesäure, 1,2 g Kaliumjodid und 2 g Dibenzo-18-Krone-6 sowie 20 g (0,22 Mol) Dimethylcarbonat in 250 g N,N-Dimethylacetamid, überführt die Mischung in einen mit PTFE ausgekleideten Autoklaven, erhitzt unter Rühren auf 110 °C und läßt 18 Stunden reagieren.
Das Reaktionsprodukt enthält laut GC kein Ausgangsmaterial und lediglich < 1 % 3-Methoxy-2,4,5-trifluorbenzoesäuremethylester. Überschüssiges Lösungsmittel wird über eine kurze Vigreux-Kolonne abdestilliert, der verbleibende, ölige Rückstand fraktioniert destilliert und die erhaltene Substanz mit 300 g heißem Wasser ausgerührt.
Man erhält 37,6 g (0,183 Mol) 3-Hydroxy-2,4,5-trifluorbenzoesäuremethylester (91 % Ausbeute; Reingehalt nach GC > 99 %).

### Beispiel 4

Man löst unter Rühren 57,6 g (0,3 Mol) 3-Hydroxy-2,4,5-trifluorbenzoesäure, 0,75 g Kaliumjodid und 1,4 g 4-Dimethylaminopyridin in 250 g Dimethylcarbonat, überführt die Mischung in einen mit PTFE ausgekleideten Autoklaven, erhitzt unter Rühren auf 170 °C und läßt 8 Stunden reagieren (Stickstoffvordruck bei 20 °C: 0,2 MPa; erreichter Enddruck 3,7 Mpa).
Das Reaktionsprodukt enthält laut GC keinen 3-Hydroxy-2,4,5-trifluorbenzoesäuremethylester.
Nach destillativer Abtrennung des Lösungsmittels und fraktionierter Destillation des verbleibenden Sumpfproduktes gehen bei 3 Torr (100 bis 102 °C Kopftemperatur) 58,0 g (0,264 Mol) 3-Methoxy-2,4,5-trifluorbenzoesäuremethylester als farbloses Öl über (88 % Ausbeute, Reingehalt 95 %).

## Patentansprüche

1. Verfahren zur Herstellung von 3-Hydroxy-2,4,5-trifluorbenzoesäurealkylester und/oder 3-Alkoxy-2,4,5-trifluorbenzoesäurealkylester, dadurch gekennzeichnet, daß man 3-Hydroxy-2,4,5-trifluorbenzoesäure bei 80 bis 200 °C, gegebenenfalls in Anwesenheit eines Katalysators, mit einem Dialkylcarbonat umsetzt.

2. Verfahren nach Anspruch 1, dadurch gekennzeichnet, daß man die Umsetzung bei 120 bis 195 °C, insbesondere 135 bis 190 °C durchführt.

3. Verfahren nach Anspruch 1 oder 2, dadurch gekennzeichnet, daß man zur Herstellung von 3-Hydroxy-2,4,5-trifluorbenzoesäurealkylester die Umsetzung bei 80 bis 165, insbesondere 120 bis 165, bevorzugt 135 bis 160 °C durchführt.

4. Verfahren nach Anspruch 1 oder 2, dadurch gekennzeichnet, daß man zur Herstellung von 3-Alkoxy-2,4,5-trifluorbenzoesäurealkylester die Umsetzung bei 145 bis 200, insbesondere 145 bis 195, bevorzugt 150 bis 190 °C durchführt.

5. Verfahren nach einem oder mehreren der Ansprüche 1 bis 4, dadurch gekennzeichnet, daß man als Katalysator eine aus einem Metalljodid und einem Alkylierungskatalysator bestehende Mischung einsetzt.

6. Verfahren nach einem oder mehreren der Ansprüche 1 bis 5, dadurch gekennzeichnet, daß man als Alkylierungskatalysator 4-Dimethylaminopyridin oder ein auf einem festen Träger fixiertes 4-Dimethylaminopyridin, insbesondere 4-Dimethylaminopyridin einsetzt.

7. Verfahren nach einem oder mehreren der Ansprüche 1 bis 5, dadurch gekennzeichnet, daß man als Metalljodid Kaliumjodid, Natriumjodid, Rubidiumjodid oder Cäsiumjodid, insbesondere Kaliumjodid einsetzt.

8. Verfahren nach einem oder mehreren der Ansprüche 1 bis 7, dadurch gekennzeichnet, daß man einen Phasentransferkatalysator zusätzlich als Co-Katalysator einsetzt.

9. Verfahren nach einem oder mehreren der Ansprüche 1 bis 8, dadurch gekennzeichnet, daß man einen Kronenether, insbesondere 18-Krone-6 oder Dibenzo-18-Krone-6 als Phasentransferkatalysator einsetzt.

10. Verfahren nach einem oder mehreren der Ansprüche 1 bis 9, dadurch gekennzeichnet, daß man das Metalljodid und gegebenenfalls den Phasentransferkatalysator in einer Menge von 0,1 bis 5, insbesondere 0,25 bis 2 Mol%, bezogen auf 3-Hydroxy-2,4,5-trifluorbenzoesäure, einsetzt.

11. Verfahren nach einem oder mehreren der Ansprüche 1 bis 10, dadurch gekennzeichnet, daß man je Mol Metalljodid den Alkylierungskatalysator in einer Menge von 1 bis 5, insbesondere 2 bis 3 Mol einsetzt.

12. Verfahren nach einem oder mehreren der Ansprüche 1 bis 11, dadurch gekennzeichnet, daß man je Mol 3-Hydroxy-2,4,5-trifluorbenzoesäure 1 bis 100, insbesondere 1,5 bis 50, bevorzugt 5 bis 30 Mol Dialkylcarbonat einsetzt.

13. Verfahren nach einem oder mehreren der Ansprüche 1 bis 12, dadurch gekennzeichnet, daß man ein Dialkylcarbonat mit 1 bis 8, insbesondere 1 bis 4 Kohlenstoffatomen je Alkylgruppe einsetzt.

14. Verfahren nach einem oder mehreren der Ansprüche 1 bis 13, dadurch gekennzeichnet, daß man Dimethylcarbonat, Diethylcarbonat, Di-n-propylcarbonat oder Di-n-butylcarbonat als Dialkylcarbonat einsetzt.

15. Verfahren nach einem oder mehreren der Ansprüche 1 bis 14, dadurch gekennzeichnet, daß man Dimethylcarbonat oder Diethylcarbonat, insbesondere Dimethylcarbonat als Dialkylcarbonat einsetzt.

16. Verfahren nach einem oder mehreren der Ansprüche 1 bis 15, dadurch gekennzeichnet, daß man ein inertes Lösungsmittel einsetzt.

17. Verfahren nach einem oder mehreren der Ansprüche 1 bis 16, dadurch gekennzeichnet, daß man einen aliphatischen Kohlenwasserstoff, einen aromatischen Kohlenwasserstoff, einen chlorierten aromatischen Kohlenwasserstoff, ein dipolar aprotisches Lösungsmittel oder Mischungen dieser Lösungsmittel als inertes Lösungsmittel einsetzt.

18. Verfahren nach einem oder mehreren der Ansprüche 1 bis 17, dadurch gekennzeichnet, daß man Hexan, Heptan oder Octan als aliphatischen Kohlenwasserstoff, Toluol oder Xylol als aromatischen Kohlenwasserstoff, Chlorbenzol, Dichlorbenzol oder Chlortoluol als chlorierten aromatischen Kohlenwasserstoff und/oder Tetrahydrofuran, Sulfolan, Dioxan oder N,N-Dimethylacetamid als dipolar aprotisches Lösungsmittel einsetzt.

## Claims

1. A process for preparing alkyl 3-hydroxy-2,4,5-trifluorobenzoate and/or alkyl 3-alkoxy-2,4,5-trifluorobenzoate, which comprises reacting 3-hydroxy-2,4,5-trifluorobenzoic acid with a dialkyl carbonate at 80 to 200°C, in the absence or presence of a catalyst.

2. The process as claimed in claim 1, wherein the reaction is carried out at 120 to 195°C, in particular 135 to 190°C.

3. The process as claimed in claim 1 or 2, wherein the reaction for preparing alkyl 3-hydroxy-2,4,5-trifluorobenzoate is carried out at 80 to 165, in particular 120 to 165, preferably 135 to 160, °C.

4. The process as claimed in claim 1 or 2, wherein the reaction for preparing alkyl 3-alkoxy-2,4,5-trifluorobenzoate is carried out at 145 to 200, in particular 145 to 195, preferably 150 to 190, °C.

5. The process as claimed in one or more of claims 1 to 4, wherein the catalyst used is a mixture comprising a metal iodide and an alkylation catalyst.

6. The process as claimed in one or more of claims 1 to 5, wherein the alkylation catalyst used is 4-dimethylaminopyridine or a 4-dimethylaminopyridine fixed on a solid support, in particular 4-dimethylaminopyridine.

7. The process as claimed in one or more of claims 1 to 5, wherein the metal iodide used is potassium iodide, sodium iodide, rubidium iodide or cesium iodide, in particular potassium iodide.

8. The process as claimed in one or more of claims 1 to 7, wherein a phase transfer catalyst is additionally used as co-catalyst.

9. The process as claimed in one or more of claims 1 to 8, wherein a crown ether, in particular 18-crown-6 or dibenzo-18-crown-6, is used as phase transfer catalyst.

10. The process as claimed in one or more of claims 1 to 9, wherein the metal iodide and the phase transfer catalyst, if used, are used in an amount of 0.1 to 5, in particular 0.25 to 2, mol %, relative to 3-hydroxy-2,4,5-trifluorobenzoic acid.

11. The process as claimed in one or more of claims 1 to 10, wherein the alkylation catalyst is used in an amount of 1 to 5, in particular 2 to 3, mol per mole of metal iodide.

12. The process as claimed in one or more of claims 1 to 11, wherein 1 to 100, in particular 1.5 to 50, preferably 5 to 30, mol of dialkyl carbonate are used per mole of 3-hydroxy-2,4,5-trifluorobenzoic acid.

13. The process as claimed in one or more of claims 1 to 12, wherein a dialkyl carbonate having 1 to 8, in particular 1 to 4, carbon atoms per alkyl group is used.

14. The process as claimed in one or more of claims 1 to 13, wherein dimethyl carbonate, diethyl carbonate, di-n-propyl carbonate or di-n-butyl carbonate is used as the dialkyl carbonate.

15. The process as claimed in one or more of claims 1 to 14, wherein dimethyl carbonate or diethyl carbonate, in particular dimethyl carbonate, is used as the dialkyl carbonate.

16. The process as claimed in one or more of claims 1 to 15, wherein an inert solvent is used.

17. The process as claimed in one or more of claims 1 to 16, wherein an aliphatic hydrocarbon, an aromatic hydrocarbon, a chlorinated aromatic hydrocarbon, a dipolar aprotic solvent or mixtures of these solvents are used as the inert solvent.

18. The process as claimed in one or more of claims 1 to 17, wherein hexane, heptane or octane is used as the aliphatic hydrocarbon, toluene or xylene is used as the aromatic hydrocarbon, chlorobenzene, dichlorobenzene or chlorotoluene is used as the chlorinated aromatic hydrocarbon and/or tetrahydrofuran, sulfolane, dioxane or N,N-dimethylacetamide is used as the dipolar aprotic solvent.

## Revendications

1. Procédé de préparation du 3-hydroxy-2,4,5-trifluorobenzoates d'alkyle et/ou du 3-alcoxy-2,4,5-trifluorobenzoates d'alkyle, caractérisé en ce qu'on fait réagir l'acide 3-hydroxy-2,4,5-trifluorobenzoïque avec un carbonate de dialkyle, à une température de 80°C à 200 °C, éventuellement en présence d'un catalyseur.

2. Procédé selon la revendication 1, caractérisé en ce que la réaction a lieu à une température de 120 à 195 °C, en particulier de 135 à 190 °C.

3. Procédé selon la revendication 1 ou 2, caractérisé en ce que la réaction, pour la préparation du 3-hydroxy-2,4,5-trifluorobenzoate d'alkyle, a lieu à une température de 80 à 165 °C, en particulier de 120 à 165 °C, de préférence de 135 à 160 °C.

4. Procédé selon la revendication 1 ou 2, caractérisé en ce que la réaction a lieu pour la préparation de 3-alcoxy-2,4,5-trifluorobenzoate d'alkyle à une température de 145 à 200 °C, en particulier de 145 à 195 °C, de préférence de 150 à 190 °C.

5. Procédé selon une ou plusieurs des revendications 1 à 4, caractérisé en ce qu'on utilise comme catalyseur un mélange comprenant un iodure de métal et un catalyseur d'alkylation.

6. Procédé selon une ou plusieurs des revendications 1 à 5, caractérisé en ce qu'on utilise comme catalyseur d'alkylation la 4-diméthylaminopyridine ou une 4-diméthylaminopyridine fixée sur un support solide, en particulier la 4-diméthylaminopyridine.

7. Procédé selon une ou plusieurs des revendications 1 à 5, caractérisé en ce qu'on utilise comme iodure de métal l'iodure de potassium, l'iodure de sodium, l'iodure de rubidium ou l'iodure de césium, en particulier l'iodure de potassium.

8. Procédé selon une ou plusieurs des revendications 1 à 7, caractérisé en ce qu'on utilise en plus un catalyseur de transfert de phase comme cocatalyseur.

9. Procédé selon une ou plusieurs des revendications 1 à 8, caractérisé en ce qu'on utilise un éther couronne, en particulier le 18-couronne-6 ou le dibenzo-18-couronne-6 comme catalyseur de transfert de phases.

10. Procédé selon une ou plusieurs des revendications 1 à 9, caractérisé en ce qu'on utilise l'iodure de métal et éventuellement le catalyseur de transfert de phases dans une quantité de 0,1 à 5, en particulier de 0,25 à 2 % en moles par rapport à l'acide 3-hydroxy-2,4,5-trifluorobenzoïque.

11. Procédé selon une ou plusieurs des revendications 1 à 10, caractérisé en ce qu'on utilise le catalyseur d'alkylation dans des quantités de 1 à 5, en particulier de 2 à 3 moles, par mole d'iodure de métal.

12. Procédé selon une ou plusieurs des revendications 1 à 11, caractérisé en ce qu'on utilise le carbonate de dialkyle dans des quantités de 1 à 100 moles, en particulier de 1,5 à 50 moles, de préférence de 5 à 30 moles, par mole d'acide 3-hydroxy-2,4,5-trifluorobenzoïque.

13. Procédé selon une ou plusieurs des revendications 1 à 12, caractérisé en ce qu'on utilise un carbonate de dialkyle avec de 1 à 8, en particulier de 1 à 4 atomes de carbone pour chaque groupe alkyle.

14. Procédé selon une ou plusieurs des revendications 1 à 13, caractérisé en ce qu'on utilise le carbonate de diméthyle, le carbonate de diéthyle, le carbonate de di-n-propyle ou le carbonate de di-n-butyle comme carbonate de dialkyle.

15. Procédé selon une ou plusieurs des revendications 1 à 14, caractérisé en ce qu'on utilise comme carbonate de dialkyle le carbonate de diméthyle ou le carbonate de diéthyle, en particulier le carbonate de diméthyl.

16. Procédé selon une ou plusieurs des revendications 1 à 15, caractérisé en ce qu'on utilise un solvant inerte.

17. Procédé selon une ou plusieurs des revendications 1 à 16, caractérisé en ce qu'on utilise comme solvant inerte un hydrocarbure aliphatique, un hydrocarbure aromatique, un hydrocarbure aromatique chloré, un solvant aprotique dipolaire ou un mélange de ces solvants.

18. Procédé selon une ou plusieurs des revendications 1 à 17, caractérisé en ce qu'on utilise l'hexane, l'heptane ou l'octane comme hydrocarbure aliphatique, le toluène ou le xylène comme hydrocarbure aromatique, le chlorobenzène, le dichlorobenzène ou le chlorotoluène comme hydrocarbures aromatiques chlorés et/ou le tétrahydrofuranne, le sulfolane, le dioxanne ou le N,N-diméthylacétamide comme solvant dipolaire aprotique.
